Europäisches Patentamt

European Patent Office

Office européen des brevets

(11) Publication number: **0 282 234**
A1

# EUROPEAN PATENT APPLICATION

(21) Application number: 88301868.1

(22) Date of filing: 03.03.88

(51) Int. Cl.⁴: **A61B 5/00** , G01N 21/17

(30) Priority: 03.03.87 AU 69660/87
29.12.87 AU 6115/87

(43) Date of publication of application:
**14.09.88 Bulletin 88/37**

(84) Designated Contracting States:
**AT BE CH DE ES FR GB GR IT LI LU NL SE**

(71) Applicant: **Dowling, Elizabeth May**
**431 Lennard Street Daniella**
**Perth Western Australia 6062(AU)**

(72) Inventor: **Dowling, Elizabeth May**
**431 Lennard Street Daniella**
**Perth Western Australia 6062(AU)**

(74) Representative: **Allam, Peter Clerk et al**
**LLOYD WISE, TREGEAR & CO. Norman**
**House 105-109 Strand**
**London WC2R 0AE(GB)**

(54) **Optoacoustic Spectroscopy.**

(57) A device and method for detecting and measuring the concentration of a substance in a sample by optoacoustic spectroscopy in which light of selected discrete frequencies is directed onto the sample and the acoustic response of the sample is detected. The frequencies are selected according to the optoacoustic spectrum of the substance under test to distinguish that substance from others. A laser (6,74,54) is used as the light source and is controlled to emit the light in pulses and a piezoelectric device (7,56,76) is used to detect the acoustic response. Light of several frequencies may be used by using several lasers or a frequency tunable laser and emitting the pulses substantially simultaneously. The device and method are particularly suited for detecting the presence of substances in vivo, e.g. detecting glucose in the bloodstream, and avoids the need to puncture the skin and may be constructed so that it can be worn by the patient but they are also suited for in-vitro testing of substances in which case the device may be made personally portable for use on the desk-top or adapted to monitor a continuous process by continuously taking and testing samples from the main process line.

Fig.7.

## OPTOACOUSTIC SPECTROSCOPY

This invention relates generally to devices for measuring the concentration of substances in samples by opto-or photoacoustic spectroscopy.

Opto-or photoacoustic spectroscopy are techniques used to identify and analyse various substances in solution or a liquid medium or gases solids. They rely on the fact that due to the nature of the molecules of a substance, they absorb light energy only at specific frequencies. The actual molecular composition and structure determines which frequencies are absorbed and which are not and each different substance absorbs light energy at a unique set of frequencies. Because each substance, by definition, has a different molecular structure, the pattern of frequencies at which light is absorbed can identify that substance. Furthermore the strength of the absorption depends on how much of the substance is present (the concentration). When absorption of light occurs, the molecules absorbing the light are heated and thus can generate an acoustic shock-wave. This shock-wave is picked up by an acoustic sensor and used to indicate that light was absorbed. It should be noted, however, that the heating effect is very small and very fast. It is possible to deduce from the acoustic signal how much light, of the frequency projected, was absorbed and thus the concentration, in the illuminated area, of the substance under test.

In practice a sample is not continuously illuminated but instead is either illuminated by pulses of light or by an intensity modulated light beam. The use of pulsed light has become popularly to be known as optoacoustic spectroscopy and the use of a modulated light beam as photoacoustic spectroscopy but in the description and the appended claims the term optoacoustic spectroscopy will be used to encompass both techniques.

Now, many urgent and non-urgent medical procedures are based on an accurate diagnosis of a patient's condition and frequently, the diagnosis is dependent on pathological tests. These often take the form of blood tests or urine tests. A sample of blood is typically drawn off and sent to a laboratory for analysis, where it is optically examined, and mixed with reagents which show the presence and concentration of substances which form the basis of the medical practitioner's diagnosis and treatment.

Further, many medical conditions require frequent monitoring of blood to adjust drug dosages to the disease condition. The most result dependent disease treatment is possibly diabetes. Insulin dependent diabetics who are seriously ill may require blood tests every hour. Normally, insulin dependent diabetics will have to monitor their own blood glucose level four times a day for two days every week.

Hitherto, all basic blood tests have been dependent on taking a blood sample, and subjecting that sample to analysis outside the patient's body. This basic fact creates discomfort to the patient and is a source of potential inaccuracies; there is no totally painless means of drawing off blood. Portable devices such as glucometers can be used to perform bed-side analysis, using a reagent strip but a finger "lance" is used to obtain a drop of blood and this is painful, and would wake many sleeping patients. This procedure, if repeated every half-hour, day and night on a patient in a critical condition, deprives them of the ability to gain respite in sleep.

More complex pathological tests must be done in a laboratory. Samples must be transported to and from locations in a hospital and so the doctors must wait, often for hours, before a result is known.

Urinology is also an important part of Pathology. Urine monitoring is more frequently performed in routine hospital practice, yet the technology used is even less efficient than that used to perform blood tests. The procedures are very labour-intensive:

a) The specimen is collected from the patient.

b) When the staff have time, basic tests are performed with reagent strips for approximations of glucose, ketones, acidity, etc.

c) Samples which indicate further attention is desired, or those from patients which require detailed analysis are placed in sterile containers and labelled.

d) The labelled specimens are eventually picked up by a hospital orderly and transported to the laboratory. Deterioration of the sample can obscure some results, this is directly related to the time-lag. In General Practice, this time lag can be a matter of days. The sample often must be refrigerated overnight. The level of contaminating organisms may multiply at room temperature this can confuse or invalidate test results.

e) Upon arrival in the laboratory, trained technicians will use expensive equipment and considerable time evaluating each sample.

f) The results will be recorded on a standard form, which must be processed to find its way back to the ward file, or to the patient records in administration. The patients physician must then be notified of the result if it is abnormal, and the doctor in turn must notify the patient.

This is clearly a time and labour consuming task. Furthermore the equipment used in the urinology laboratory is very expensive and even the computerised reagent equipment is fairly labour-intensive.

In science and industry there are countless situations in which a detailed analysis of the chemical constituents of liquids is required. When monitoring the dispersion of pollutants in water for example, numerous samples. must be taken, transported to a laboratory, and have a battery of tests performed on them. In the manufacturing industry liquids are used which must have chemical concentrations which fall within a narrow range of variation. Frequent samples must be drawn off, and the plant adjusted accordingly. Food processing typically deals with large volumes of fluids which must conform to given standards, e.g. salinity, alcohol, or sugar content. The time taken to perform these tests by conventional means creates a greater variation in the manufacturing process, and adds to the cost of production.

Hitherto, chemical testing has been largely dependent on reagent based tests, and methods of spectroscopy which involve large, expensive equipment with associated specialist staff and facilities. Samples have to be taken, sometimes interrupting the production process, and a significant time delay occurs between sampling and data being made available to reset the plant. These problems mean that in general, only tests which are absolutely necessary are performed, and those tests which are vital, are done at the maximum interval. This means a distinct variation in the purity of the end product, as a cycle of inaccuracy is accepted and "built in" to the finished item. For the time between each test result being completed the production engineer is in effect, working blind. Considerable waste is incurred if an unexpected variable alters the constituents significantly, between test procedures. Samples taken in the field often become corrupted over time. If there is a long lag between sampling and testing, the liquid may have chemically changed by the time it is actually analysed.

According to the invention there is provided a device for detecting the concentration of a substance in a sample by optoacoustic spectroscopy including a sensor unit including a light source for emitting light into the sample and an acoustic sensor for receiving the acoustic response from the sample under test wherein the light source is adapted to emit light of one frequency or of a plurality of discrete frequencies, the frequency or frequencies being set in accordance with the optoacoustic spectrum of the substance under test.

It is preferable for the light to be emitted in pulses rather than continuously and in the case that the sample is being illuminated by light at several frequencies, the pulses at the different frequencies should all be emitted substantially simultaneously, preferably within about 10 micro seconds of each other.

Conveniently the light source is a semiconductor laser and it is housed in a housing containing an acoustic sensor, for example a piezoelectric device, for receiving the acoustic response from the sample under test. The acoustic sensor may be disposed around the light source or alternatively can comprise a plurality of physically separate sensors disposed on the housing with their output connected together.

Preferably the sensor further includes a photodiode and photocell to allow reflectance and, in some embodiments, transmittance measurements, to be carried out on the sample and the device may also include a temperature sensor, e.g. a fast acting semiconductor device, to allow the temperature of the sample to be measured.

According to a further aspect the present invention provides a device for the measurement of the concentration of substances in a body fluid which is adapted to carry out the measurement in vivo by the fact that it carries a light source for emitting light into the body under test and an acoustic sensor for detecting the acoustic response of the body to measure the concentration of said substance by optoacoustic spectroscopy.

The invention further provides a method for detecting a substance in a body fluid including the step of detecting the substance in vivo by optoacoustic spectroscopy.

Preferably the device is of such a size and shape that it is personally portable and it includes means for attaching it to the human or animal body e.g. a bracelet. The device may also include means for monitoring the heart beat of the body, this may be done either by monitoring the signal from the piezoelectric device used in the optoacoustic spectroscopy test or by using a separate photodiode and photocell monitoring the light to reflectance of the body at a frequency which is absorbed by blood. Furthermore, the circuitry controlling the energization of the light sources for the optoacoustic spectroscopy may include means for timing the energization of the light sources in relation to the heart beat.

The invention further provides a device for the sensing of a substance in the human or animal body wherein the substance is sensed by optoacoustic spectroscopy and the illuminating of the body for the test is controlled in timed relation to the heartbeat of the body under test.

Preferably the light source for the optoacoustic movement is adapted to emit light at one or a plurality of different discrete frequencies, the frequency or frequencies being set in relation to the optoacoustic spectrum of a substance under test.

Preferably the devices are provided with means for recording the results of the optoacoustic spectroscopy test and also means for recording the time in relation to the heart beat at which the test was made.

Conveniently there is provided a microprocessor which controls the energisation of the various diodes, and interprets the signals received. The signals may be calibrated and displayed, or relayed to other control or recording machinery. Thus the device may give a relatively fast and/or continuous display of the concentration of certain blood constituents.

Preferably the microprocessor will automatically diagnose failure of the laser diodes and display a "fault" alert.

Preferably the laser or lasers are tuned, not only to a characteristic frequency of the substance under test but also to operate in the laser therapeutic window of the skin of the body under test.

With this aspect of the invention a wide range of substances may be tested such as the constituents of blood, urine, tear duct fluid, bone marrow, cerebro-spinal fluids, faeces and nasal secretions. It may also be used to detect the presence of drugs, gases, toxins, pollutants and metals and to detect spectroscopic response patterns of overall disease conditions such as arthritis, lupis, porphyrias diabetes, cancers, AIDS and anaemia and to detect the presence of bacteria and provide information as to their population density, stage of development, type or susceptibility to antibiotics.

According to a further aspect of the present invention there is provided a device for the in-vitro testing of substances using the technique of optoacoustic spectroscopy. In this device the sensors may be disposed around a cavity which receives a container for a sample e.g. a test tube. Preferably the cavity includes a sample sensor for sensing the physical presence of the sample and means, responsive to the sample sensor for moving the other sensors in the housing into position for the test, conveniently, against the side of the test tube. Alternatively, the device may be constructed so that the sensors are resiliently supported and the sample tube is a tight fit in the housing in contact with the sensors. Preferably the device is a self contained, portable unit so that it may be used on a desktop. The device may be provided with a variety of sensors apart from the laser and piezoelectric sensor required for optoacoustic spectroscopy just as in the device described above for in-vivo measurements. Thus the light transmittance, reflectance and temperature of a sample may be measured.

The signal processing and laser drive circuitry may be generally the same as in the above aspects of the present invention. It is envisaged that the desk-top in-vitro device could be provided with a plurality of lasers firing at different frequencies so that substances with complex optoacoustic spectra may be tested for.

Where a plurality of the lasers are provided it is preferably for the lasers to be arranged offset from the piezoelectric sensors i.e. so that no laser is directly opposite a piezoelectric sensor. This means that no laser will fire directly into a piezoelectric sensor.

With this aspect of the present invention a wide range of drugs, licit, and illicit, may be tested for as well as metals, hormones and steroids which possess a clear spectroscopic signature. The device may also be used to test for parasitic infections in body fluids, and overall disease conditions and bacteria as mentioned in connection with the first aspect described above.

To detect the presence of substances which may be elusive to direct spectroscopic detection, the devices according to the present invention may include a measurement cycle which involves the introduction of one or more reagents into the specimen. The chemical reactions which take place with reagents serve to "amplify" the presence of target substances to an extraordinary degree, working in concert with spectroscopic techniques, this will enhance the versatility of reagents by allowing measurements at lower concentrations, or earlier phases of the reaction.

According to yet a further aspect of the present invention there is provided a device for taking samples from a continuous process and subjecting them to optoacoustic spectroscopy. Preferably the device comprises a sample tube connected to the main process line, the sample tube may include means for smoothing the flow of sample through the tube so as to reduce the noise in a sample. These means may be baffles or alternatively the fluid may be stopped temporarily in the sample tube while the test is being carried out. Preferably an array of laser diodes and piezoelectric transducers are provided around the sample tube for performing the optoacoustic spectroscopy. The signal processing and laser control parts of the apparatus are generally similar to those described in connection with the second embodiments of the invention. The analysis circuitry of the invention may be interfaced to the main controlling units of the continuous process so that a continuous control loop is set up.

The invention may thus function "in-line" with a manufacturing process, whereupon the results of the analysis can be used to electronically adjust units of the plant which govern the relevant chemical concentrations. Used in this way, it can provide instant feedback at selected points of the manufacturing process, and can thus ensure fully automated quality control. This embodiment of the invention is particularly suitable for use in the oil industry, beer making and many chemical processes.

In embodying any or all of the above aspects the device may be adapted so it can easily be configured to test for different substances. Clearly different substances may require lasers firing at different frequencies and so this adaptability may be achieved by providing the lasers and piezoelectric devices in a plug-in module which may also contain any circuitry which is specific to the substance with which that module is intended for use. Furthermore, any of the devices may be adapted e.g. by programming the microprocessor to take a reading or number of readings at nominated intervals.

With the above described "in vitro" testing devices fibre optic devices may be used to extend the sensors into environments which would be dangerous to the operator or device. Thus the laser diode outputs would be fed into fibre optic cables leading to the sample and light microphones used to pick up the acoustic response and feed it back via fibre optics to the analysis and processing circuitry.

Furthermore, where in any of the above aspects, a plurality of light sources are used then it is possible to test different parts of the sample by arranging and controlling the light sources to form a phased array.

According to yet a further aspect of the present invention there is provided a method of detecting the concentration of a substance in a sample by optoacoustic spectroscopy comprising the steps of emitting light into the sample and detecting the acoustic response of the sample including the step of selecting the frequency of light emitted into the sample to be at least one of a plurality of discrete frequencies set in accordance with the optoacoustic absorption spectrum of the substance under test.

Preferably the light of a plurality of discrete frequencies emitted in the sample, preferably in the form of pulses emitted substantially simultaneously with each other preferably within about 10 microseconds of each other.

The fact that the pulses are emitted substantially simultaneously gives rise to a number of advantages. Firstly, there is insuffcient time for diffusion of the sample between the various firing paths (this is important where the sample might be affected by the laser light). Seconly the effects of sample deterioration with time are mitigated since all of the measurements are taken within a short time of each other and thirdly acoustic interference between the different lasers is also reduced or eliminated.

Thus with each of the aspects of the present invention the presence and concentration of certain substances in a sample are measured by the method of optoacoustic spectroscopy. The frequency of light used is usually in the I.R. range, e.g. 700 to 2500 nm. The predetermined frequency or frequencies are decided according to the absorption spectrum of the substance to be measured. In most cases, such as the measurement of glucose in blood, it is necessary to measure the acoustic response at several frequencies in order to differentiate them from other substances and so either a tunable laser capable of being set to a number of discrete frequencies or a number of lasers emitting light of different frequencies are required. The testing of two or more frequencies also allows background noise to be eliminated by subtracting the results of the tests from one another. It is preferred that several lasers are used so that they may all be controlled to emit a pulse of light virtually simultaneously (within microseconds of each other) so that the measurement at each of the frequencies is performed at virtually the same time. This is particularly advantageous in the case of samples such as urine which deteriorate with time and can be changed by the laser light itself. Thus, in the present invention a complete continuum of frequencies is not scanned. Instead light is only emitted at that frequency or those frequencies which are needed to detect the substance under test and distinguish it from other substances. The acoustic response is then processed electronically to give a reading of the concentration of that substance in the sample, i.e. an interpreted result, not a spectrogram. This approach means that the present invention can provide a device which is easy to use and which can be used by people having relatively little skill and virtually no prior knowledge of optoacoustic spectroscopy is needed and thus that the accurate technique of optoacoustic spectroscopy can be used much more widely and conveniently than was previously possible.

Furthermore, the use of a pulsed light source not only has the advantages of increasing the signal to noise ratio and allowing, if required, a depth profile (i.e. an indication of concentration at different depths in the sample) of a given substance.

The present invention will be further described by way of non-limitative example with reference to the accompanying drawings in which:-

Figure 1 shows a first embodiment of the present invention;

Figure 2 shows a second embodiment of the present invention;

Figure 3 is a diagramatic side view of a third embodiment of the present invention;

Figure 4 is a diagramatic top view of the embodiment of figure 3;

Figure 5 is a diagramatic view of a fourth embodiment of the present invention;

Figure 6 is a schematic diagram of the electronic circuitry of one embodiment of the present invention; and

Figure 7 is a further schematic diagram of the electronic circuitry of one embodiment of the present invention.

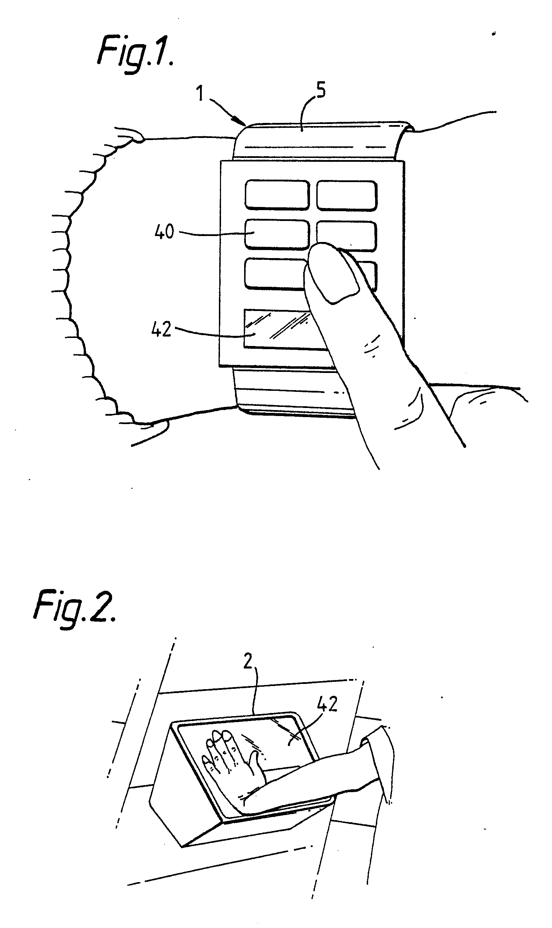

Figures 1 and 2 show devices for in-vivo measurement of substances in the blood. In both embodiments a sense head containing both the laser for emitting light and the acoustic sensor for detecting the acoustic response caused by the light is held against the skin so that spectroscopic readings of the blood under the surface of the skin may be carried out. In the embodiment shown in figure 1 the sense head is mounted together with processing and display circuitry on a bracelet 3 and so the device may be worn continuously. This is particularly useful for people such as diabetics who benefit from frequent or continuous monitoring of blood glucose levels. The embodiment shown in figure 2 has the sense head mounted in a console against which the patient places part of the body e.g. the hand.

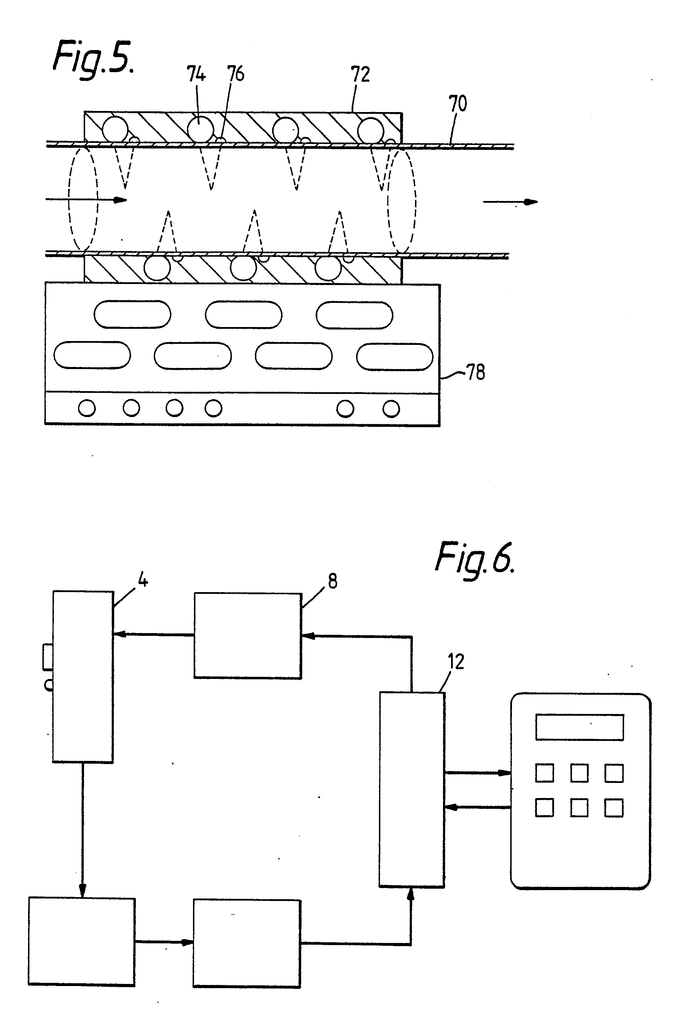

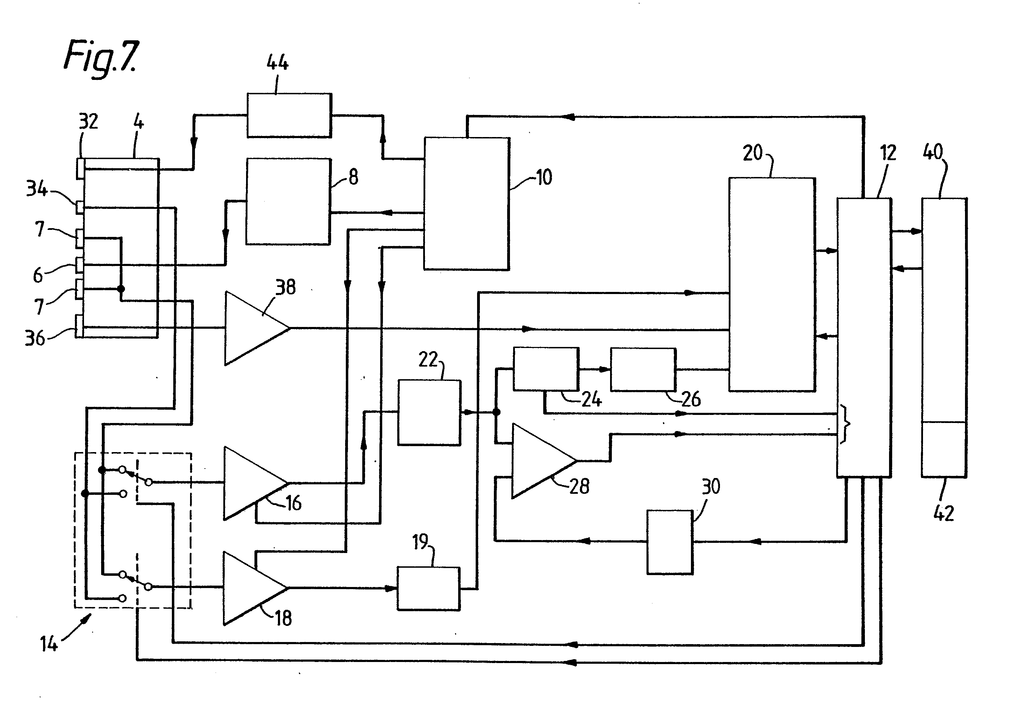

The device's sense head and its controlling circuitry are shown schematically in figures 6 and 7 and it can be seen that the sense head 4 includes a semiconductor laser 6 and piezoelectric transducers 7. In use the semiconductor laser is energised to emit a pulse of laser light, the light energy is absorbed by molecules in the blood which heat up and this heating up causes acoustic waves which are picked up by the piezoelectric transducers 7. The semiconductor laser 6 is controlled by a laser drive unit 8 and controller 10 connected to the main controlling microprocessor 12 of the unit. The signal from the piezoelectric sensors 7 is fed via a switching unit 14 into one of two signal paths each containing a wide band gated amplifier 16, 18. The output from amplifier 18 is made available for spectroscopic analysis and processing by analyser 19 (which may be part of microprocessor 12 or be separate circuitry) and fed as spectroscopic data via analogue to digital converter 20 to the microprocessor 12. The other signal path, via amplifier 16, is used to provided a signal representative of the heart beat of the patient. The signal is filtered by a low pass filter 22 to remove noise and is fed via a peak-detect circuit 24 and sample-and-hold circuit 26 to the analogue-to-digital convertor 20 and thence to micro-processor 12. The output of the sample-and-hold circuit is the representative of the patient's pulse strength and the output of the peak detect circuit 24, which is also fed directly to the microprocessor 12, is representative of the peak of the heart beat. The controller 10, is used to gate amplifiers 16 and 18 which increases the signal-to-noise ratio. The heartbeat signal may be used to time the energisation of the semiconductor laser 6. It is therefore possible for the laser to be fired at a particular point in the heart beat and/or to know exactly at what point during the heart beat the measurement has been made.

The output from the low pass filter 22 is also fed to a threshold setting circuit consisting of comparator 28, microprocessor 12 and digital to analogue convertor 30. This allows one to set the level at which the heart beat signal is provided.

In an alternative embodiment the laser is a frequency tunable laser and a controller is provided so that the laser frequency may be adjusted and measurements may be made at a number of frequencies. The laser may also have specially configured lenses on it to achieve special penetration effects by shaping the light beam such as control over the depth of penetration, the intensity, selective filtration of wavelenghts, diffusion at damage-vulnerable levels of the skin of the patient, concentration at the depth of tissue under study and the quantification of the zone of activation for the purposes of obtaining calibration data.

In the two embodiments of the invention shown in figures 1 and 2 the sense head 4 is also provided with other sensors for making a variety of other measurements on the body. As can be seen from figure 7 the sense head includes a semiconductor light source 32 driven by driver 44, photodetector 34 and a fast-acting temperature transducer 36. The semiconductor light source and photodetector can be used to measure the amount of blood flowing beneath the surface of the skin by measuring the reflectance of the body. The frequency of the semiconductor light source is chosen so that the light will be absorbed by blood and thus the signal at the photodetector will depend on the amount of blood beneath the skin. The signal from the photodetector 34 is also supplied to the switching unit 14 so that it can be processed by the signal processing circuitry already described above. This signal can be used to measure the volume of blood beneath the skin, heart beat or simply to indicate that the device has been correctly placed against the body of the patient. It can thus be used to control the energization of other parts of the circuitry so that they are only energized when the device is in contact with the skin. It also provides an indication of whether the device is being used correctly and they whether the optoacoustic measurement is likely to be meaningful. The fast acting temperature transducer 36 is preferably of the semiconductor type and is used to measure the body temperature of the patient. The output signal from the transducer 36 is supplied to an amplifier/conditioner 38 and then to the analogue-to-digital convertor 24 for input into the microprocessor 12 as a series of 8 or 10 bit data words.

The fact that the volume of blood beneath the skin can be measured means that the blood pressure of the patient can also be deduced. The invention thus also provides a way of testing the blood pressure of a patient without any compression of the patient's body.

The microprocessor 12 is connected to operator controls 40 and a display 42, e.g. an LCD display as shown in figures 1 and 2.

The microprocessor 12 is programmed to control the device and to calibrate the results in to standard units. The results are stored in a memory (not shown separately) capable of storing about 1000 readings as well as displayed on the displayed and the microprocessor may be programmed to average results for a number of spectroscopic cycles.

The use of a microprocesor also allows the device to perform some degree of self-calibration. Clearly the absolute measurements coming from the sense head will be affected by a number of factors, e.g. skin thickness, which will vary from patient to patient. The measurement may be calibrated, however, by looking at signals from other substantces which have known values and which are stored in the memory of the microprocessor.

The piezoelectric transducers 7 in the sense head may comprise several separate transducers as shown or may be, for instance, an annular transducer disposed around the semiconductor laser. It is a feature of piezo-electric transducers that they can be arranged to give a close, direct acoustic coupling to the body being measured. This is true of both the in vivo measurement under discussion and of the in vito measurement discussed below.

The device may also include an interface (not shown) for allowing the data to be transferred to a device controlling the administration of a substance to the patient. For instance the device can be used to monitor the blood glucose level of a diabetic and this information transferred to circuitry controlling a pump administering insulin to the patient.

The device described above in relation to figure 1 is particularly suitable for measuring the glucose level in blood and therefore provides a very convenient device for use by diabetics who need to frequently monitor their blood sugar level. At present the blood sugar level is monitored by puncturing the skin and using a reagent strip - this is messy and, especially if it needs to be done frequently, is painful. The present device can be worn continuously and read-outs taken at any time. Furthermore the results of the tests can be stored in the device and there is no question of the results being altered either intentionally or unintentionally by the patient. It is also possible for the device to give a reading representative of the confidence of the spectroscopic result as it can monitor, amongst other things, its own battery level, the condition of the laser diodes, whether the device was placed correctly in close contact with the skin, the temperature of the patient and the point in the heat beat at which the measurement was made. It may also store one or more profiles of standard measurements for comparison with the actual result so that, again, an indication of the confidence of the result can be obtained.

The fact that the spectroscopic reading can be related to the heart-beat means that the signal-to-noise ratio of the reading can be improved. The signal-to-noise ratio is further improved by the use of a pulse laser, rather than continuous illumination of the surface, and the use of a pulse laser allows a depth profile of a concentration of substance being measured to be picked up. The depth profile can easily be built up by timing the interval between the arrival of the acoustic wave and the end of the laser pulse.

The above description has been made in relation to a device for measuring blood glucose levels, however the device is also suitable for measuring other substances in the blood. If necessary the sense head may be provided with a number of lasers each operating at a different frequency and some or all of the lasers may be tunable so that simultaneous measurements at a number of frequencies can be carried out. This can be necessary if the optoacoustic spectrum of the substance under test is complex. In the version of the device with a number of lasers it is possible for the lasers to be provided in a plug-in module also including any circuitry specific to those lasers or to the processing of the signal from that substance so that the device may easily be configured to measure one of a number of different substances. Thus the device may be programmed to test for different antibodies in the blood e.g. Aids antibodies etc. The device therefore provides the possiblity of screening large numbers of people or animals in a very short time and thus could be very important in providing improved healthcare programmes.

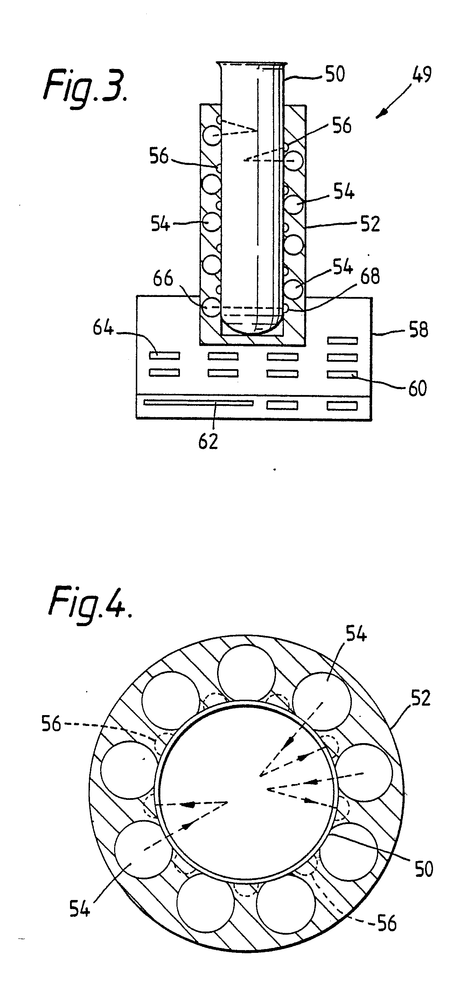

The present invention is also applicable to the in-vitro testing of samples and the figures 3, 4 and 5 show embodiments of the invention suitable for this.

The embodiment shown in figures 3 and 4 is a desk top device for the testing of samples contained in, for instance, a test tube 50. The device comprises a support 52 for the test tube including a cavity for receiving the test tube and an array of radiators and receivers disposed around the inside surface of the cavity. These radiators and receivers comprise lasers 54 and piezoelectric transducers 56. As mentioned above in testing for some substances it may be necessary to monitor the response of the sample to light of a number of frequencies and so a plurality of lasers and piezoelectric transducers are provided. Each of the lasers is tuned to one of the frequencies which it is necessary to test at, and, optionally, some or all of the lasers may also be tunable so that they can test at more than one frequency. It will be seen from figures 3 and 4 that the piezoelectric transducers are offset, i.e. they are not positioned directly opposite one another, so that the light from one laser is not directed straight into any of the piezoelectric transducers.

The support 52 is mounted on a base unit 58 which includes a display 60, user-operable controls 64 and optionally a printer 62. The base unit also contains the power supply circuitry, signal processing circuitry and the micro-processor.

The electronic circuitry controlling the device is generally similar to that of figures 6 and 7 except that the laser driver is adapted to drive a plurality of lasers and signal processing circuitry for each of the piezoelectric devices is provided.

In use the semiconductor lasers of the frequency which it is necessary to test at are all driven to emit a pulse of laser light simultaneously or within microseconds of each other. This means that all of the measurements at the various different frequencies are taken at the same time or close to one another which is important if the sample is deteriorating with time (urine, for example, deteriorates fairly quickly) and also means that in samples in which impurities are formed by action of the laser on the sample there is not time for the impurities to diffuse from the firing path of one laser to that of another. Where measurement is made on a moving or flowing sample, such as a fluid, e.g. blood, the rapidity of measurement ensures that all measurement ensures that all measurements are effectively on the same sample.

The lasers may have specially configured lenses on them to achieve special penetration effects by shaping the light beam analogous to those in the in-vivo device described above.

In a further embodiment of this device it is possible for measurements of several different substances to be made on one sample. This is achieved by providing lasers adapted to emit light at all of the different frequencies required to test at the characteristic frequencies of each sample or by providing a sufficient number of tunable lasers and programming the apparatus so that first the lasers fire at all of the light frequencies required for one particular substance and the acoustic response measured, then all the light frequencies for the next substance and so on. In a further, alternative, embodiment, rather than providing many different lasers or tunable lasers to cover a number of substances, the support containing the lasers and piezoelectric transducers may be in the form of a plug-in module containing a sufficient number of lasers to fire at all of the frequencies required for one particular substance or set of substances. The module may also contain any parts of the control and signal processing circuitry which are specific to detection of a particular substance. In the embodiment of Figure 3 and 4 the base unit holds the circuitry which is common to the testing of all relevant substances. The modules may include electronic circuitry to process the signals from the sensors and provide them in a common, predetermined format to the base unit which then displays the result. It is envisaged, therefore, that a doctor or hospital ward etc could be provided with on desk top base unit and a "library" of plug-in modules for testing a variety of substances and so be able to test samples from patients quickly and easily and be presented with an immediately understandable result.

The desk-top device shown in figures 3 and 4 may, like the in-vivo device, also include other sensors. It may therefore have a temperature transducer for measuring the temperature of the sample and a photodiode 66 with one photo cell opposite it to allow the light transmittance to be measured and a further photo cell next to it to allow the light reflectance to be measured The processing circuitry may also include circuitry to monitor the condition of the laser diodes and indicate to the user if any of these are faulty.

The unit is also provided with a pressure sensitive device at the base of the cavity in the support 62 to sense the physical presence of a test tube and actuate a mechanism to bring the laser diodes and piezoelectric sensors into firm contact with the side of the test tube.

An alternative embodiment has a sense head with no moving parts where the sensors are resiliently supported in the housing and the test tube is squeezed into the housing by hand.

The device may also be used to test non-medical samples such as water (for pollutants etc) liquid food components, industrial fluids, gases in liquids, agricultural products, plant and animal, for pesticides and may even be used for testing solid products by mixing them with a suitable fluid to provide a solution or suspension. The device shown includes a printer that can provide a printout of the results and the device also includes a memory for storing the results.

The device thus provides a convenient desk top unit which may be used to analyse such fluids as blood, urine, tear duct fluid, bone marrow, cerebro-spinal fluids, faeces and nasal secretion. It will give an immediate, accurate, result in terms of concentration of substance in the sample and does not require any particular skill on the part of the user. Furthermore the fact that readings at all of the frequencies require to detect a particular substance are made at the same time or within a very short time of each other means that the results are accurate. The device is also flexible in that it may be programmed to look for any number of substances or sets of substances as long as the particular optoacoustic spectrum for that substance is known. The type of substances which may be searched for are any substance relevant to pathology or urinology and listings of such substances may be found in the Merck Manual of Diagnosis and Therapy, 14th Edition, pages 2, 184 to 2,201 inclusive (appendix 1). Further substances which the device is effective to test for are drugs, legally or illegally administered, drugs which are licit or illicit, gases, toxins, pollutants, metals and other substances which may be tested for by optoacoustic spectroscopy.

In a typical application of this embodiment of the invention a unit may be placed on each ward of a hospital so that urine (and other body fluid) testing may be done in situ. The results may then be entered immediately onto the patient's records. An alternative embodiment is provided with an interface to allow the data to be transferred to a computerised record, for instance held centrally by the hospital.

A further embodiment of the invention which is particularly applicable to on-line continuous monitoring industrial processes is shown in figure 6. In this embodiment an array of lasers and piezoelectric transducers analogous to those in the figures 3 and 4 embodiments are arranged around a sample tube 70. The sample tube 70 is connected as a branch to a main process line carrying the material to be tested. A sample from the main process line is diverted into the sample tube 70, preferably in a controlled manner, so that a smooth laminar flow without acoustic noise is achieved. If noise is particularly prevalent in any given situation it may be necessary to provide baffles in the sample tube or to stop the fluid temporarily during the test.

The processing and control circuitry is housed in the body 78 of the device and is entirely analogous to circuitry 58 of the figures 3 and 4 embodiment. It may also include an interface to allow it to be connected to the apparatus controlling the main process so that a continuous control loop is provided. The interface may further allow input of data from the main controlling apparatus of the process or from other sampling points on the process line.

This device may be used to test any sample containing a substance whose optoacoustic spectrum is known and is particularly useful for monitoring the purity or viscosity of petroleum products, the concentration of liquids in the food industry e.g. salt, sugar, contaminates, wine, milk, alcohol, oils, suspensions or solutions from many other larger volume liquid processes.

It is again envisaged that the lasers and samples/substance specific circuitry could be provided within a plug-in module or be programmable so that the device can easily be configured to monitor different substances.

It will be appreciated that the device is particularly easy to install on existing plant by simply attaching the sample tube to appropriate points of the main process line and the sensors are set and the circuitry is programmed according to the particular application required.

It is envisaged that where semi-conductor components are used for the sensing functions of the device, all of the components could be formed on a single microchip. This would mean that the device could be made more compact and would be more rugged in use.

Mention has been made of measuring a sample at a plurality of wavelengths. To obtain a quantitative measurement it is considered preferable to measure a given substance at a peak of its optoacoustic response and at an off-peak wavelength to provide a second measurement which is combined with the peak measurement to give the quantitative value. The off-peak reference allows for laser power variability and other opto-acoustic transmission variables. By way of example an aqueous solution of glucose can be measured at 1580 nm and 1300 nm, the latter being the reference value. User laser pulse lengths at the two wavelengths of say 0.5microseconds with a pulse interval of say 10 microseconds, the acoustic data resulting from each pulse can be acquired and stored for processing.

## Claims

1. A device for the measurement of the concentration of substances in a body fluid characterised in that it is adapted to carry out the measurement in-vivo by the fact that it carries a light source (6,54,74) for emitting light into the body under test and an acoustic sensor (7,56,76) for detecting the acoustic response of the body to measure the concentration of said substance by optoacoustic spectroscopy.

2. A device according to claim 1 characterised in that the light source (6,54,74) is adapted to emit light at at least one of a plurality of discrete frequencies set in accordance with the optoacoustic spectrum of the substance under test.

3. A device according to claim 2 characterised in that the light source (6,54,74) is adapted to emit light at each of said plurality of discrete frequencies and in that means (8) are provided to control the energization of the light source to emit the light in pulses emitted substantially simultaneously.

4. A device according to claim 3, characterised in that the pulses are emitted within ten microseconds of each other.

5. A device for detecting the concentration of a substance in a sample by optoacoustic spectroscopy including a sensor unit (4,72,52) including a light source (6,54,74) for emitting light into the sample and an acoustic sensor (7,56,76) for receiving the acoustic response from the sample under test characterised in that the light source (6,54,74) is adapted to emit light of one frequency or of a plurality of discrete frequencies, the frequency or frequencies being set in accordance with the optoacoustic spectrum of the substance under test.

6. A device according to claim 5 characterised by a light driver (8) for controlling the energization of the light source (6,54,74) to emit the light in pulses.

7. A device according to claim 6 characterised in that the light driver (8) is adapted to control the light source (6,54,74) to emit the light pulses substantially simultaneously.

8. A device according to claim 7 characterised in that the pulses are emitted within 10 micro seconds of each other.

9. A device according to claim 5, 6, 7 or 8 characterised in that the light source (6,54,74) is adapted to emit light at a plurality of different discrete frequencies, the frequencies being set in relation to the optoacoustic spectrum of the substance under test.

10. A device according to any one of claims 5 to 9 characterised in that the sensor unit forms a sense head (4) having a sense surface on which the light source (6) and acoustic sensor (7) are disposed whereby, in use, the device may be positioned with the sense surface in contact with a human, animal or plant body to perform an in-vivo measurement of the substance under test.

11. A device according to claim 1, 2, 3 or 10 characterised in that the device is of such a size and shape that it is personally portable.

12. A device according to claim 11 characterised in that the device includes attachment means (3) for attaching it to the human or animal body.

13. A device according to claim 12 characterised in that said attachment means (3) comprises a bracelet.

14. A device according to any one of claims 10 to 13 and adapted for use with the human or animal body characterised by means for monitoring the heart beat of the body.

15. A device according to claim 14 characterised in that said means (7) comprises the acoustic sensor and processing means (16,22,24,26) for processing the signal from the acoustic sensor to derive a signal relating to the heart beat of the body.

16. A device according to claim 14 or 15 characterised by timing means (12) for timing the energization of the light source (6) in relation to the heart beat.

17. A device according to claim 14, 15 or 16 characterised by recording means (12) for recording the time in relation to the heart beat at which the optoacoustic measurement is taken.

18. A device according to any one of claims 5 to 9 characterised in that the sensor unit is mounted in a housing (52) for receiving the sample under test.

19. A device according to claim 18 characterised in that the housing (52) defines a cavity into which the sample is placed, the light source (54) and acoustic sensor (56) are mounted on the inside surface of the cavity.

20. A device according to claim 18 or 19 characterised in that the housing (52) includes a sample sensor for sensing the presence of the sample and means, responsive to the sample sensor for moving the sensor unit into position for the test.

21. A device according to claim 18, 19 or 20 characterised in that the device is a self-contained, portable unit.

22. A device according to any one of claims 5 to 9 characterised in that it includes a sample tube (70) having an inlet and an outlet and through which a sample may pass, the sensor unit (72) being disposed adjacent to the sample tube (70) so that the light source (74) directs light into the sample tube.

23. A device according to claim 22 characterised in that the sensor unit (72) is disposed surrounding the sample tube (70).

24. A device according to claim 22 or 23 characterised in that the sample tube (70) includes means for reducing acoustic noise in the sample.

25. A device according to claim 24 characterised in that the means are adapted to smooth the flow of the sample through the tube (70).

26. A device according to claim 24 or 25 characterised in that the means comprise baffles in the sample tube (70).

27. A device according to claim 24 characterised in that the means comprise valves for closing the inlet and/or outlet of the sample tube (70).

28. A device according to any one of the preceding claims characterised in that the light source (6,54,74) is a semi-conductor laser.

29. A device according to any one of the preceding claims characterised in that the acoustic sensor (7,56,76) is a piezo-electric device.

30. A device according to any one of the preceding claims characterised in that the acoustic sensor (7,56,76) is a microphone.

31. A device according to any one of the preceding claims characterised in that the acoustic sensor (7,56,76) is disposed on the sensor unit and surrounding the light source (6,54,74).

32. A device according to any one of the preceding claims characterised in that the acoustic sensor (7,56,76) comprises a plurality of physically separate sensors disposed on the sensor unit with their outputs connected together.

33. A device according to any one of the preceding claims characterised by an illumination source (66) and an illumination detector (68) so disposed to allow the light transmittance of the sample to be measured.

34. A device according to any one of the preceding claims characterised by an illumination source (32) and an illumination detector (38) so disposed on the sensor unit to allow the light reflectance of the sample to be measured.

35. A device according to any one of the preceding claims characterised in that the sensor unit further includes a temperature sensor (36) mounted thereon.

36. A device according to claim 35 characterised in that the temperature sensor (36) is a semiconductor device.

37. A device according to any one of the preceding claims characterised by memory means (12) for storing the results of the optoacoustic measurement.

38. A device according to any one of the preceding claims characterised by display means (42) for displaying the result of the optoacoustic measurement.

39. A device according to any one of the preceding claims characterised in that the light source (6,54,74) is mounted in a removeable module to be connected, in use, to the device and a plurality of modules are provided each mounting a light source (6,54,74) adapted to emit light at of least one of a plurality of discrete frequencies set in accordance with the optoacoustic spectrum of a substance to be tested.

40. A device according to claim 39 characterised in that the modules contain electronic circuitry programmed to process the acoustic signal from the substance under test to produce an output in a predetermined format and circuitry for transferring that output to the device.

41. A method of detecting the concentration of a substance in a sample by optoacoustic spectroscopy comprising the steps of emitting light into the sample and detecting the acoustic response of the sample characterised by the step of selecting the frequency of light emitted into the sample to be at least one of a plurality of discrete frequencies set in accordance with the optoacoustic absorption spectrum of the substance under test.

42. A method according to claim 41 characterised by emitting a pulse of light at each of a plurality of said discrete frequencies into the sample.

43. A method according to claim 42 characterised in that said pulses are emitted substantially simultaneously.

44. A method according to claim 43 characterised in that said pulses are emitted within ten micro seconds of each other.

45. A device for the sensing of a substance in the human or animal body characterised by means (6,7,54,56,74,76) for sensing the substance by optoacoustic spectroscopy and the illumination of the body for the test is controlled in timed relation to the heartbeat of the body under test.

46. A method for detecting a substance in a body fluid characterised by the step of detecting the substance in-vivo by optoacoustic spectroscopy.

47. A method according to claim 46 characterised by the step of controlling the illumination of the body for the optoacoustic spectroscopy to be in timed relation to the heartbeat of the body under test

# Fig.1.

# Fig.2.

Fig.3.

Fig.4.

*Fig.5.*

*Fig.6.*

# Fig.7.

0 282 234

# DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | JOURNAL OF PHYSICS. E. SCIENTIFIC INSTRUMENTS, vol. 16, no. 8, August 1983, pages 738-739, The Institute of Physics, London, GB; D.H. McQUEEN: "A simplified open photoacoustic cell and its applications" | 1 | A 61 B 5/00<br>G 01 N 21/17 |
| X | IDEM | 5,41 | |
| A | IDEM | 9,10,29,42,46 | |
| | --- | | |
| Y | LABORATORY PRACTICE, vol. 25, 6th June 1976, pages 377-383, London, GB; A.A. KING et al.: "Optoacoustic spectrometry for the examination of solid and semi-solid samples"<br>* Pages 378-379, section "Principle of operation of OAS for solid examples, figures; pages 381-382, section "Biochemical and biological applications" * | 1 | |
| X | --- | 5,41 | TECHNICAL FIELDS SEARCHED (Int. Cl.4)<br><br>A 61 B<br>G 01 N |
| A | | 2,9,10,42,46 | |
| | --- | | |
| X | EP-A-0 195 685 (WESTINGHOUSE ELECTRIC CORP.)<br>* Figure; page 6, line 28 - page 8, line 7 * | 5,41 | |
| A | | 1,2,9,27,40,42 | |
| | ---     -/- | | |

The present search report has been drawn up for all claims

| Place of search | Date of completion of the search | Examiner |
|---|---|---|
| THE HAGUE | 22-06-1988 | CHEN A.H. |

EPO FORM 1503 03.82 (P0401)

European Patent
Office

EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-4 200 399  (KIMBLE et al.) <br> * Figures 1,2; column 1, line 49 - column 3, line 6 * <br> --- | 5,41 | |
| A | US-A-4 200 399 <br> --- | 1,22,40 | |
| A | BIOMEDIZINISCHE TECHNIK BAND 31, Heft 7/8, July-August 1986, pages 169-174, Berlin, DE; H. SCHMIDT-KLOIBER et al.: "Die photoakustische Spektroskopie - ein geeignetes Hilfsmittel zur Analyse biologischer Proben" <br> * Pages 169-171, section "2 Der photoakustische Effekt an Festkörpen" - section "4 Beschreibung der Experiments"; Figures 1,2 * <br> --- | 1,2,5,9 ,10,19, 29,30, 37,38, 40-42 | |
| A | MEDICAL & BIOLOGICAL ENGINEERING & COMPUTING, vol. 23, no. 6, November 1985, pages 585-588, IFMBE, Stevenage, GB; P. POULET et al.: "In vivo cutaneous spectroscopy by photoacoustic detection" <br> * Abstract; pages 586-588, section "2 Photoacoustic spectrometer" - section "5 Conclusion" * <br> --- | 1,2,5, 10,12, 18,19, 30,37, 38,41, 46 | TECHNICAL FIELDS SEARCHED (Int. Cl.4) |
| A | US-A-4 533 252  (CAHEN et al.) <br> * Figures 1-3; column 1, line 67 - column 2, line 42; column 3, lines 7-50 * <br> --- | 1,2,5,9 ,10,19, 30,41, 42,46 | |
| A | EP-A-0 178 653  (FUJI PHOTO FILM CO.) <br> * Figures 1-3,6; page 6, line 27 - page 10, line 49; page 11, line 6 - page 15, line 11 * <br> ---                          -/- | 3,6,28 | |

The present search report has been drawn up for all claims

| Place of search | Date of completion of the search | Examiner |
|---|---|---|
| THE HAGUE | 22-06-1988 | CHEN A.H. |

EPO FORM 1503 03.82 (P0401)

European Patent Office

EUROPEAN SEARCH REPORT

Application Number

EP 88 30 1868

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| A | Y.-H. PAO.: "Optoacoustic Spectroscopy and Detection", 1977, pages 54-55, Academic Press Inc., New York, US; * Pages 54-55, figure 2, section "B. Sample chamber design"; page 55, lines 1-19 * | 24-26 | |
| A | US-A-4 281 645 (F.F. JöBSIS) * Figures 5-8; column 3, line 38 - column 18, line 40 * | 2,3,6,7 ,9,10, 14,16, 17,28 | |
| | | | **TECHNICAL FIELDS SEARCHED (Int. Cl.4)** |

The present search report has been drawn up for all claims

| Place of search | Date of completion of the search | Examiner |
|---|---|---|
| THE HAGUE | 22-06-1988 | CHEN A.H. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 
& : member of the same patent family, corresponding document

EPO FORM 1503 03.82 (P0401)